# EUROPÄISCHE PATENTSCHRIFT

(11) **EP 2 183 206 B1**
(45) Veröffentlichungstag und Bekanntmachung des Hinweises auf die Patenterteilung: **24.02.2016**
(21) Anmeldenummer: 08786661.2
(22) Anmeldetag: 31.07.2008
(51) Int. Cl.: C07C 29/70

(54) **VERFAHREN ZUR HERSTELLUNG VON LITHIUMALKOHOLAT-LÖSUNGEN**
PROCESS FOR THE PREPARATION OF LITHIUM ALCOHOLATE SOLUTIONS
PROCÉDÉ DE PRÉPARATION DE SOLUTIONS D'ALCOOLATE DE LITHIUM

(30) Priorität: 31.07.2007 DE 102007036275
(43) Veröffentlichungstag der Anmeldung: 12.05.2010
(73) Patentinhaber: Chemetall GmbH, 60487 Frankfurt (DE)
(72) Erfinder: DAWIDOWSKI, Dirk, 61169 Friedberg (DE); WIETELMANN, Ulrich, 61381 Friedrichsdorf (DE); RITTMEYER, Peter, 65843 Sulzbach/Taunus (DE)
(74) Vertreter: Rottmayer, Hans
(86) Internationale Anmeldenummer: PCT/EP2008/060036
(87) Internationale Veröffentlichungsnummer: WO 2009/016217

(56) Entgegenhaltungen:
- US-A- 5 276 219
- US-B1- 6 194 617
- CONRAD W. KAMIENSKI ET AL: 'Relationship between Structure and Solubility of Organic Lithium Compounds' THE JOURNAL OF ORGANIC CHEMISTRY Bd. 30, Nr. 10, 01 Oktober 1965, Seiten 3498 - 3504, XP055036107 DOI: 10.1021/jo01021a051 ISSN: 0022-3263

## Beschreibung

Gegenstand der vorliegenden Erfindung ist ein Verfahren zur Herstellung von Lithiumalkoholat-Lösungen.

Alkalimetallalkoholate werden in der organischen Synthese als Basen für die Herstellung von Zwischenprodukten und Feinchemikalien eingesetzt. Diese finden z. B. als Pharma-, Agro- sowie Geruchs- und Geschmacksstoffe Verwendung.

Lithiumalkoholate können durch Umsetzung von Organolithiumverbindungen mit Alkoholen gemäß:

ROH + R'Li → R'H + ROLi

hergestellt werden. Eine andere, kommerziell günstigere Methode geht von metallischem Lithium anstelle von Organolithiumverbindungen aus:

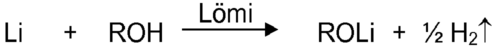

Diese Reaktion wird in der Regel in organischen Lösungsmitteln (Lömi) durchgeführt, die gegenüber Li-Metall inert sein müssen. Als solche Lösemittel eignen sich aprotische organische Verbindungen wie Ether (z. B. Diethylether, THF, Dibutylether, Ethylenglykoldialkylether), aromatische Kohlenwasserstoffe (Benzol, Toluol, Ethylbenzol, Cumol etc.) oder gesättigte Kohlenwasserstoffe (z. B. Pentan, Hexan, Heptan, Paraffinöle).

Grundsätzlich kann das Lithiummetall in geschmolzener oder fester Form vorliegen. Der Einsatz von geschmolzenem Metall erfordert jedoch wegen dessen hohen Schmelzpunktes von 180,5 °C in der Regel eine Fahrweise unter erhöhtem Druck (US 3,971,833). Nachteil dieser Technik sind demzufolge Sicherheitsrisiken wegen der extremen Reaktivität von geschmolzenem Lithium sowie die relativ hohen Investitionskosten für druckfeste Anlagen.

Wird festes Lithiummetall eingesetzt, so ist die Reaktivität insbesondere gegenüber sekundären und tertiären Alkoholen stark reduziert, d. h. die Reaktionszeiten sind häufig deutlich verlängert, was negative Auswirkungen auf die Prozesswirtschaftlichkeit hat. Um die Reaktionsgeschwindigkeit zu erhöhen, sind folgende Möglichkeiten bekannt:
- Einsatz einer feinteiligen Lithiumqualität und eines Alkoholüberschusses. So beschreibt US 5,276,219 die Herstellung von Lithium-tert-butoxid als Lösung in THF mit fein verteiltem Lithium (< 300 µm) bei Rückflusstemperaturen (ca. 66 °C) unter Verwendung eines Alkohol-Überschusses von mind. 5 bis 20 % (bzw. 100 %). Als Lithiumqualität wird handelsübliches Metall mit einem Na-Gehalt von ca. 0,4 - 0,76 % eingesetzt. Statt THF können auch andere polare Lösungsmittel (Ether oder Amine) eingesetzt werden. Vorteil des Verfahrens ist, dass klare oder einfach filtrierbare Produktlösungen entstehen. Nachteilig ist, dass die Produktlösungen mit freiem Alkohol verunreinigt sind.
- Einsatz eines Überschusses an Na-armem Li-Metall in grobstückiger Form. So beschreibt US 5,583,269 (EP 721445) eine Methode zur Herstellung von Lithium-tert-butoxid dadurch gekennzeichnet, dass Lithiummetall mit einem Na-Gehalt von max. 0,1 % in Abformung von min. 1 cm³/Stück mit einem tertiären Alkohol in einem Lösungsmittel ausgewählt aus Ethern oder Kohlenwasserstoffen umgesetzt wird. Das Molverhältnis Lithium:Alkohol liegt dabei zwischen 2:1 und 10:1 und die Reaktionstemperatur zwischen 34,6 und 100 °C. Der Nachteil dieses Verfahrens besteht darin, dass nur bei Einsatz eines genügend hohen Überschusses an Lithium wirtschaftlich akzeptable Reaktionszeiten realisiert werden können. Das vom Überschuss-Lithium eingenommene Volumen geht von der spezifischen Produktmenge (I Produkt pro I Reaktorvolumen) ab. Außerdem bleibt beim letzten Ansatz einer Kampagne relativ viel Lithium übrig, das aufwändig entsorgt oder mit z. B. Wasser desaktiviert werden muss.

Ein weiterer Nachteil der beiden oben beschriebenen Verfahren zeigt sich, wenn die Synthese in THF durchgeführt wird, ein Lösungsmittel, das in der US 5,276,219 "zwingend vorgeschrieben" wird: Lithiummetall reagiert mit THF unter Ringöffnung, wodurch ein unlöslicher, äußerst fein verteilter Feststoff entsteht, der die Produktlösungen praktisch unfiltrierbar macht. Dadurch wird die Abtrennung überschüssigen Lithiums sehr erschwert bzw. unmöglich gemacht. Außerdem stören die unlöslichen Trübstoffe häufig bei nachfolgenden Anwendungen in der organischen Synthese. Des Weiteren ist die Löslichkeit von Lithium-alkoholaten in THF sehr begrenzt, z. B. beträgt die Sättigungskonzentration von LTB nur ca. 20 %.

Aus dem Dokument US 6,194,617 B1 ist die Herstellung von Lithium-tert.-alkoxiden-Lösungen in etherischen oder Kohlenwasserstoff Lösemitteln oder deren Mischungen bekannt, die einen Natriumgehalt von weniger als 10 ppm aufweisen. Dazu wird Lithiummetall, welches einen Natriumgehalt von weniger als 0,1 Gew.-% aufweist, in Blockform mit einem Volumen von mindestens 1 cm³ zur Umsetzung mit dem tert. Alkohol eingesetzt.

Weiterhin ist aus der Veröffentlichung Conrad W. Kamienski et al., J. of Org. Chem., Bd. 30, Nr. 10, S. 3498-3504 die Herstellung von Lithiumalkoholaten in Heptan bekannt. Die Herstellung erfolgt jedoch auch in diesem Fall aus zu kleinen Stücken geschnittenen Lithiummetall-Stäben mit einem Durchmesser von 1/8 inch und ohne Angabe des Natriumgehaltes im eingesetzten Lithiummetall.

Die Aufgabe der vorliegenden Erfindung besteht darin, ein Verfahren zu entwickeln, das die Nachteile des Standes der Technik überwindet, insbesondere ein Verfahren bereitzustellen, das gut filtrierbare Lösungen von Li-salzen sekundärer und tertiärer Alkohole in einem organischen Lösungsmittel liefert, die möglichst keinen oder nur max. 10 mol% an freiem Alkohol enthalten. Das Verfahren soll kurze Reaktionszeiten ermöglichen und nicht auf die Verwendung hoher Lithiumüberschüsse angewiesen sein.

Die der Erfindung zugrunde liegende Aufgabe wird erfindungsgemäß dadurch gelöst, dass tert.-Amylalkohol, tert.-Butylalkohol oder Isopropylalkohol mit Lithiummetall mit einem maximalen Na-Gehalt 0,01 % Na-Gehalt umgesetzt wird. Das Lithiummetall wird dabei in Abformungen von Lithiumpartikel <0,1 ml, besonders bevorzugt < 0,01 ml pro Stück eingesetzt. Das Lithiummetall liegt dabei im Überschuss von von 5 bis 30 %, bezogen auf die Gesamtalkoholmenge vor.

Als aprotisches Lösungsmittel werden erfindungsgemäß Hexan oder Heptan eingesetzt.

In einer bevorzugten Herstellvariante wird das Lithiummetall im aprotischen Lösungsmittel vorgelegt und unter Rühren mit dem gewünschten sekundären oder tertiären Alkohol versetzt. Die Reaktionstemperatur liegt im Allgemeinen zwischen 0 und 150 °C, bevorzugt 20 - 100 °C, besonders bevorzugt bei 30 °C bis zum Siedepunkt des verwendeten Lösungsmittels bzw. Lösungsmittelgemisches. Die Dosierzeit für den Alkohol ist von der Reaktivität des jeweiligen Alkohols ROH (im Allgemeinen fällt die Reaktivität mit der Sperrigkeit des Restes R, d. h. sekundäre Alkohole reagieren schneller als tertiäre), der Temperatur und der Oberfläche des metallischen Lithiums zum jeweiligen Zeitpunkt abhängig. Im Allgemeinen liegt die Dosierzeit zwischen 10 Minuten und 20 Stunden, bevorzugt 1 - 10 Stunden.

Es wurde überraschend gefunden, dass die notwendige Reaktionszeit vom Na-Gehalt des verwendeten Lithiums abhängt und dass die Reaktion umso schneller läuft, je geringer der Na-Gehalt ist. Dies ist für den Fachmann insofern überraschend, als Natrium im Allgemeinen das Lithium für Reaktionen z. B. in Kohlenwasserstoffen aktiviert (W.N. Smith Jr, J. Organomet. Chem. 82, 1974, 1-6). Zwar ist für den Fall des Systems Li/THF/tert-Butanol eine Beschleunigung der Reaktion durch abnehmenden Na-Gehalt bekannt (US 5,583,269), jedoch handelt es sich hier um einen ganz spezifischen Effekt, der auf die Spaltung (Ringöffnung) des 5-Ringes THF durch Lithium zurückgeht. Da nach dem vorliegenden erfindungsgemäße Verfahren keine cyclischen Ether verwendet werden, ist ein retardierender Effekt durch Natrium folgerichtig nicht zu erwarten.

Beobachtet wurde jedoch, dass die Reaktionsgeschwindigkeit zu Anfang eines Ansatzes weitgehend unabhängig vom Na-Gehalt ist, sie jedoch im Laufe der Reaktion stark abnimmt. Dieser Effekt geht darauf zurück, dass die Lithiumpartikel in Abhängigkeit vom Na-Gehalt unterschiedlich schnell und stark verklumpen. Dadurch nimmt die Li-Oberfläche ggfls stark ab, so dass extrem lange Reaktionszeiten benötigt werden. Erst beim Unterschreiten eines Na-Gehaltes von 0,01 %, findet man keine signifikante Agglomerations-/ Verklumpungstendenz mehr. Infolge dessen benötigen Syntheseansätze mit Na-armem Lithiummetall nur einen Bruchteil der Reaktionszeiten verglichen mit solchen Ansätzen, die mit "technischem Li-Metall" durchgeführt werden, d.h. solchem mit 0,2 % Na-Gehalt.

Ganz besonders geeignet ist das erfindungsgemäße Verfahren zur Herstellung von Lithium-tert-butoxid (LTB), Lithium-tert-amoxid (LTA) und Lithium-iso-propoxid(LIP) in Hexan oder Heptan-haltigen Lösungsmitteln.

## Patentansprüche

1. Verfahren zur Herstellung von Lithiumalkoholatlösungen in einem aprotischen Lösungsmittel oder Lösungsmittelgemisch, **dadurch gekennzeichnet, dass** tert.-Amylalkohol, tert.-Butylalkohol oder Isopropylalkohol mit einem Überschuss von 5 - 30 % an Lithiummetall, das einen Natriumgehalt von max. 0,01 % aufweist, umgesetzt wird, wobei das Lithiummetall in Partikeln mit einem Volumen von max. 0,1 ml vorliegt, als Lösungsmittel Hexan oder Heptan eingesetzt werden und gut filtrierbare Lösungen von Lithium-tert-amoxid, Lithium-tert-butoxid, Lithiumiso-propoxid, die möglichst keinen oder nur max. 10 mol% an freiem Alkohol enthalten, erhalten werden.

2. Verfahren gemäß Anspruch 1, **dadurch gekennzeichnet, dass** das Lithiummetall in Partikeln mit einem Volumen von max. 0,01 ml vorliegt.

3. Verfahren nach Anspruch 1 oder 2, **dadurch gekennzeichnet, dass** die Reaktion bei Temperaturen von 0 - 150 °C durchgeführt wird.

4. Verfahren nach Anspruch 3, **dadurch gekennzeichnet, dass** die Reaktion bei Temperaturen von 20 - 100 °C durchgeführt wird.

5. Verfahren nach Anspruch 4, **dadurch gekennzeichnet, dass** die Reaktion bei Temperaturen zwischen 30°C und Siedetemperatur des Lösungsmittels durchgeführt wird.

6. Verfahren gemäß einem oder mehreren der Ansprüche 1 bis 5, **dadurch gekennzeichnet, dass** die Dosierzeit für den Alkohol von 10 Minuten - 20 Stunden beträgt.

7. Verfahren nach Anspruch 6, **dadurch gekennzeichnet, dass** die Dosierzeit für den Alkohol 1 - 10 h beträgt.

## Claims

1. A process for the preparation of lithium alcoholate solutions in an aprotic solvent or solvent mixture, **characterised in that** tert-amyl alcohol, tern-butyl alcohol or isopropyl alcohol is reacted with an excess of 5 - 30 % lithium metal which has a sodium content of a maximum of 0.01 %, wherein the lithium metal is present in particles with a volume of a maximum of 0.1 ml, hexane or heptane is used as solvent, and easily filterable solutions of lithium tert-amoxide, lithium tert-butoxide, lithium isopropoxide are obtained that contain as far as possible no or only a maximum of 10 molar % free alcohol.

2. A process according to claim 1, **characterised in that** the lithium metal is present in particles with a volume of a maximum of 0.01 ml.

3. A process according to claim 1 or 2, **characterised in that** the reaction is carried out at temperatures of 0 - 150 °C.

4. A process according to claim 3, **characterised in that** the reaction is carried out at temperatures of 20 - 100 °C.

5. A process according to claim 4, **characterised in that** the reaction is carried out at temperatures between 30 °C and boiling temperature of the solvent.

6. A process according to one or more of claims 1 to 5, **characterised in that** the metering time for the alcohol amounts to 10 minutes - 20 hours.

7. A process according to claim 6, **characterised in that** the metering time for the alcohol amounts to 1 - 10 h.

## Revendications

1. Procédé de préparation de solutions d'alcoolate de lithium dans un solvant aprotique ou un mélange de solvants, **caractérisé en ce que** l'on transforme de l'alcool tert.-amylique, de l'alcool tert.-butylique ou de l'alcool isopropylique avec un surplus allant de 5 à 30 % de lithium métal qui présente une teneur en sodium d'au maximum 0,01 %, le lithium métal étant présent en particules avec un volume d'au maximum 0,1 ml, que l'on utilise comme solvant de l'hexane ou de l'heptane, et que l'on obtient des solutions facilement filtrables de tert-amoxyde de lithium, de tert-butoxyde de lithium, d'iso-propoxyde de lithium, qui ne contiennent autant que possible pas d'alcool libre ou au maximum 10 % molaire d'alcool libre.

2. Procédé selon la revendication 1, caractérisé e, ce que le lithium métal est présent en particules avec un volume d'au maximum 0,01 ml.

3. Procédé selon la revendication 1 ou 2, **caractérisé en ce que** la réaction est réalisée à des températures allant de 0 à 150°C.

4. Procédé selon la revendication 3, **caractérisé en ce que** la réaction est réalisée à des températures allant de 20 à 100 °C.

5. Procédé selon la revendication 4, **caractérisé en ce que** la réaction est réalisée à des températures comprises entre 30 °C et la température d'ébullition du solvant.

6. Procédé selon une ou plusieurs des revendications 1 à 5, **caractérisé en ce que** le temps de dosage pour l'alcool est de 10 minutes à 20 heures.

7. Procédé selon la revendication 6, **caractérisé en ce que** le temps de dosage pour l'alcool est de 1 à 10 heures.
